# EUROPÄISCHE PATENTSCHRIFT

(11) **EP 2 436 685 B1**
(45) Veröffentlichungstag und Bekanntmachung des Hinweises auf die Patenterteilung: **01.07.2015**
(21) Anmeldenummer: 11189691.6
(22) Anmeldetag: 29.11.2005
(51) Int. Cl.: C07D 475/00

(54) **Zwischenprodukte zur Herststellung von annelierten Piperazin-2-on Derivaten**
Intermediate products for producing annulated piperazine-2-on derivatives
Produits intermédiaires pour la procédé de fabrication de dérivés de pipérazine-2-ons annelés

(30) Priorität: 02.12.2004 DE 102004058337
(43) Veröffentlichungstag der Anmeldung: 04.04.2012
(62) Teilanmeldung aus: 05823799.1
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Duran, Adil, 88400 Biberach (DE); Linz, Guenter, 88441 Mittelbiberach (DE)
(74) Vertreter: Simon, Elke Anna Maria

(56) Entgegenhaltungen:
- WO-A-96/36597
- WO-A-03/020722
- TENBRINK R E ET AL: "ANTAGONIST, PARTIAL AGONIST, AND FULL AGONIST IMIDAZO1,5-AQUINOXALINE AMIDES AND CARBAMATES ACTING THROUGH THE GABAA/BENZODIAZEPINE RECEPTOR", JOURNAL OF MEDICINAL CHEMISTRY, AMERICAN CHEMICAL SOCIETY. WASHINGTON, US, Bd. 37, 1994, Seiten 758-768, XP001012790, ISSN: 0022-2623

## Beschreibung

Die Erfindung betrifft Zwischenprodukte für Verfahren zur Herstellung von annelierten Piperazin-2-on Derivaten der allgemeinen Formel (I) 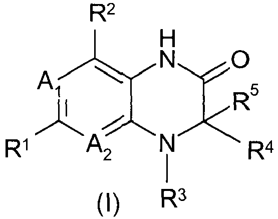 wobei die Reste R¹ bis R⁵ die in den Ansprüchen und der Beschreibung genannten Bedeutungen haben, insbesondere ein Verfahren zur Herstellung von 7,8-Dihydro-5H-pteridin-6-on Derivaten.

### Hintergrund der Erfindung

Pteridinon-Derivate sind als Wirkstoffe mit antiproliferativer Wirkung aus dem Stand der Technik bekannt. WO 03/020722 beschreibt die Verwendung von Dihydropteridinonderivaten zur Behandlung von Tumorerkrankungen sowie Verfahren zu deren Herstellung.

7,8-Dihydro-5H-pteridin-6-on Derivate der Formel (I) stellen wichtige Zwischenprodukte in der Synthese solcher Wirkstoffe dar. Für ihre Herstellung wurden bisher Reduktionsmethoden auf Nitroverbindungen der unten beschriebenen Formel (II) angewandt, welche zu stark gefärbten Produktgemischen führten und aufwendige Aufarbeitungs- und Reinigungsschritte erforderlich machten.

WO 96/36597 beschreibt die katalytische Hydrierung von Nitroverbindungen mittels Edelmetallkatalysatoren unter Zusatz einer Vanadiumverbindung, wobei als Endprodukte freie Amine, aber keine Laktame, offenbart werden.

TenBrink et al., (J. Med. Chem. 37, 1994, 758-768) offenbart die Synthese verschiedener Quinoxalinamide durch Reduktion von an Benzolringen gebundener Nitrogruppen, welche von einer Ringzyklisierung gefolgt ist.

Es ist die Aufgabe der vorliegenden Erfindung Zwischenprodukte für ein verbessertes Verfahren zur

Herstellung von Verbindungen der Formel (I), insbesondere von 7,8-Dihydro-5H-pteridin-6-on Derivaten, bereitzustellen.

### Detaillierte Beschreibung der Erfindung

Die vorliegende Erfindung löst die vorstehend genannte Aufgabe für das im folgenden beschriebene Syntheseverfahren für Verbindungen der Formel (I).

Es handelt sich um ein Verfahren zur Herstellung von Verbindungen der allgemeinen Formel I 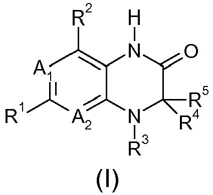 worin
- R¹: ein Rest ausgewählt aus der Gruppe bestehend aus Chlor, Fluor, Brom, Methansulfonyl, Ethansulfonyl, Trifluormethansulfonyl, para-Toluolsulfonyl, CH₃S(=O)- und PhenylS(=O)-
- R²: Wasserstoff oder C₁-C₃-Alkyl,
- R³: Wasserstoff oder ein Rest ausgewählt aus der Gruppe bestehend aus gegebenenfalls substituiertem C₁-C₁₂-Alkyl, C₂-C₁₂-Alkenyl, C₂-C₁₂-Alkinyl und C₆-C₁₄-Aryl, oder
ein Rest ausgewählt aus der Gruppe bestehend aus gegebenenfalls substituiertem und/oder verbrücktem C₃-C₁₂-Cycloalkyl, C₃-C₁₂-Cycloalkenyl, C₇-C₁₂-Polycycloalkyl, C₇-C₁₂-Polycycloalkenyl, C₅-C₁₂-Spirocycloalkyl und gesättigtes oder ungesättigtes C₃-C₁₂-Heterocycloalkyl, das 1 bis 2 Heteroatome enthält,
- R⁴, R⁵: gleich oder verschieden, Wasserstoff oder gegebenenfalls substituiertes C₁-C₆-Alkyl, oder
- R⁴ und R⁵: gemeinsam eine 2- bis 5-gliedrige Alkylbrücke, die 1 bis 2 Heteroatome enthalten kann, oder
- R⁴ und R³ oder R⁵ und R³: gemeinsam eine gesättigte oder ungesättigte C₃-C₄-Alkylbrücke, die gegebenenfalls 1 Heteroatom enthalten kann,
und
A₁ und A₂ -N=
bedeuten,
wobei eine Verbindung der Formel II 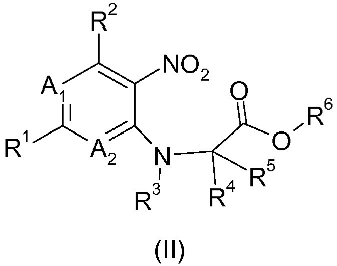 worin
R¹-R⁵ und A₁, A₂ die angegebene Bedeutung aufweisen und
R⁶ C₁-C₄-Alkyl bedeutet,
   a) in Anwesenheit eines Hydrierkatalysators mit Wasserstoff hydriert wird und
   b) eine Kupfer-, Eisen- oder Vanadium-Verbindung zugegeben wird,
      wobei die Schritte a) und b) gleichzeitig oder nacheinander erfolgen können.

Geeignet sind die beanspruchten Zwischenprodukte auch für ein Verfahren, wobei die Hydrierung der Verbindung der Formel II direkt in Anwesenheit des Hydrierkatalysators und der Kupfer-, Eisen- oder Vanadium-Verbindung zur Verbindung der Formel I durchgeführt wird.

Besonders geeignet sind die beanspruchten Zwischenprodukte der Formel (III) für ein Verfahren, wobei nach dem ersten Hydrierungsschritt a) zunächst das Zwischenprodukt der Formel III erhalten wird, das gegebenenfalls isoliert werden kann, 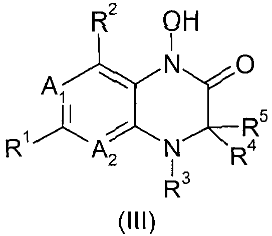 und anschließend in Gegenwart von einem Hydrierkatalysator und einer Kupfer-, Eisen- oder Vanadium-Verbindung zu einer Verbindung der Formel I weiterreduziert wird

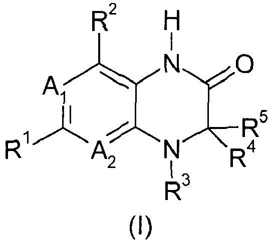

Bevorzugt ist ein Verfahren, wobei der Hydrierkatalysator ausgewählt ist aus der Gruppe bestehend aus Rhodium, Ruthenium, Iridium, Platin, Palladium und Nickel vorzugsweise Platin, Palladium und Raney-Nickel. Insbesondere bevorzugt ist Platin. Platin kann in metallischer Form oder oxidierter Form als Platinoxid auf Trägem wie z.B. Aktivkohle, Siliziumdioxid, Aluminiumoxid, Calziumcarbonat, Calziumphosphat, Calziumsulfat, Bariumsulfat, Titandioxid, Magnesiumoxid, Eisenoxid, Bleioxid, Bleisulfat oder Bleicarbonat und gegebenenfalls zusätzlich dotiert mit Schwefel oder Blei verwendet werden. Bevorzugtes Trägermaterial ist Aktivkohle, Siliziumdioxid oder Aluminiumoxid.

Bevorzugte Kupfer-Verbindungen sind Verbindungen in denen Kupfer die Oxidationsstufen I oder II einnimmt, beispielsweise die Halogenide des Kupfers wie z.B. CuCl, CuCl₂, CuBr, CuBr₂, CuI oder CuSO₄. Bevorzugte Eisen-Verbindungen sind Verbindungen in denen Eisen die Oxidationsstufen II oder III einnimmt, beispielsweise die Halogenide des Eisens wie z.B. FeCl₂, FeCl₃, FeBr₂, FeBr₃, FeF₂ oder andere Eisenverbindungen wie z.B. FeSO₄, FePO₄ oder Fe(acac)₂.
Bevorzugte Vanadium-Verbindungen sind Verbindungen in denen Vanadium die Oxidationsstufen 0, II, III, IV oder V einnimmt, beispielsweise anorganische oder organische Verbindungen oder Komplexe wie z.B. V₂O₃, V₂O₅, V₂O₄ Na₄VO₄, NaVO₃, NH₄VO₃, VOCl₂, VOCl₃, VOSO₄, VCl₂, VCl₃, Vanadiumoxobis(1-phenyl-1,3-butandionat), Vanadiumoxotriisopropoxid, Vanadium(III)acetylacetonat [V(acac)₃]oder Vanadium(IV)oxyacetylacetonat [VO(acac)₂]. Besonders bevorzugt ist Vanadium(IV)oxyacetylacetonat [VO(acac)₂].

Die Kupfer-, Eisen- oder Vanadium-Verbindung kann wahlweise entweder direkt zu Beginn der Hydrierung eingesetzt werden oder nach Bildung der Zwischenstufe der Formel (III).

Weiterhin bevorzugt ist ein Verfahren, worin die Menge an zugesetztem HydrierKatalysator zwischen 0.1 und 10 Gewichts-% bezogen auf die eingesetzte Menge der Verbindung der Formel (II) liegt.

Weiterhin bevorzugt ist ein Verfahren, worin die Menge an eingesetzter Kupfer-, Eisen- oder Vanadium-Verbindung zwischen 0.01 und 10 Gewichts-% bezogen auf die eingesetzte Menge der Verbindung der Formel (II) liegt.

Weiterhin bevorzugt ist ein Verfahren, worin die Reaktion durchgeführt wird in einem Lösungsmittel ausgewählt aus der Gruppe bestehend aus: Dipolaren, aprotischen Lösungsmitteln, beispielsweise Dimethylformamid, Dimethylacetamid, N-Methylpyrrolidinon, Dimethylsulfoxid oder Sulfolan; Alkoholen, beispielsweise Methanol, Ethanol, 1-Propanol, 2-Propanol, die verschiedenen isomeren Alkohole des Butans und Pentans; Ethern, beispielsweise Diethylether, Methyl-tert.-butylether, Tetrahydrofuran, 2-Methyltetrahydrofuran, Dioxan oder Dimethoxyethan; Estern, beispielsweise Ethylacetat, 2-Propylacetat oder 1-Butylacetat; Ketonen, beispielsweise Aceton, Methylethylketon oder Methylisobutylketon; Carbonsäuren, beispielsweise Essigsäure; Apolaren Lösungsmitteln, beispielsweise Toluol, Xylol, Cyclohexan oder Methylcyclohexan, sowie aus Acetonitril, Methylenchlorid und Wasser.
Die Lösungsmittel können auch als Gemische eingesetzt werden.

Weiterhin bevorzugt ist ein Verfahren, worin die Reaktionstemperatur zwischen 0 °C und 150.°C, vorzugsweise zwischen 20°C und 100 °C liegt.

Weiterhin bevorzugt ist ein Verfahren, worin der Wasserstoffdruck 1 bar bis 100 bar beträgt.

Der Gegenstand der Erfindung ist eine Verbindung der Formel (III) 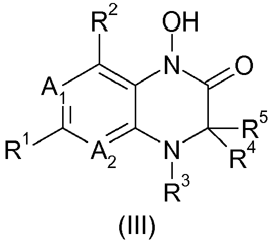 worin R¹ bis R⁵ diein den Ansprüchen angegebene Bedeutung aufweisen können und wobei A₁ und A₂ gleich -N= bedeuten.

Die Aufarbeitung der Umsetzungen erfolgt nach gängigen Methoden z.B. über extraktive Reinigungsschritte oder Fäll- und Kristallisations-Prozeduren.

Die erfindungsgemäßen Verbindungen können in Form der einzelnen optischen Isomeren, Mischungen der einzelnen Enantiomeren, Diastereomeren oder Racemate, in

Form der Tautomere sowie in Form der freien Basen oder der entsprechenden Säureadditionssalze mit Säuren - wie beispielsweise Säureadditionssalze mit Halogenwasserstoffsäuren, beispielsweise Chlor- oder Bromwasserstoffsäure, oder organische Säuren, wie beispielsweise Oxal-, Fumar-, Diglycol- oder Methansulfonsäure, vorliegen.

Als Alkylgruppen sowie Alkylgruppen, welche Bestandteil anderer Reste sind, werden verzweigte und unverzweigte Alkylgruppen mit 1 bis 12 Kohlenstoffatomen bevorzugt 1 - 6, besonders bevorzugt 1-4 Kohlenstoffatomen bezeichnet, beispielsweise werden genannt: Methyl, Ethyl, Propyl, Butyl, Pentyl, Hexyl, Heptyl, Octyl, Nonyl, Decyl und Dodecyl. Sofern nicht anders genannt, sind von den vorstehend genannten Bezeichnungen Propyl, Butyl, Pentyl, Hexyl, Heptyl, Octyl, Nonyl, Decyl und Dodecyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc.

In den vorstehend genannten Alkylgruppen können gegebenenfalls ein oder mehrere Wasserstoffatome durch andere Reste ersetzt sein. Beispielsweise können diese Alkylgruppen durch Fluor substituiert sein. Es können gegebenenfalls auch alle Wasserstoffatome der Alkylgruppe ersetzt sein.

Als Alkylbrücke werden, soweit nicht anders angegeben, verzweigte und unverzweigte Alkylgruppen mit 2 bis 5 Kohlenstoffatomen, beispielsweise Ethylen, Propylen-, Isopropylen-, n-Butylen, iso-Butyl, sec. Butyl und tert.-Butyl etc. Brücken bezeichnet. Besonders bevorzugt sind Etyhlen, Propylen- und Butylen-Brücken. In den genannten Alkylbrücken können gegebenenfalls 1 bis 2 C-Atome durch ein oder mehrere Heteroatome ausgewählt aus der Gruppe Sauerstoff, Stickstoff oder Schwefel ersetzt sein.

Als Alkenylgruppen (auch soweit sie Bestandteil anderer Reste sind) werden verzweigte und unverzweigte Alkylengruppen mit 2 bis 12 Kohlenstoffatomen, bevorzugt 2 - 6 Kohlenstoffatomen, besonders bevorzugt 2 - 3 Kohlenstoffatomen betrachtet, soweit sie mindestens eine Doppelbindung aufweisen. Beispielsweise werden genannt: Ethenyl, Propenyl, Butenyl, Pentenyl etc. Sofern nicht anders genannt, sind von den vorstehend genannten Bezeichnungen Propenyl, Butenyl etc. sämtliche der möglichen isomeren Formen umfaßt. Beispielsweise umfaßt die Bezeichnung Butenyl 1-Butenyl, 2-Butenyl, 3-Butenyl, 1-Methyl-1-Propenyl, 1-Methyl-2-Propenyl, 2-Methyl-1-Propenyl, 2-Methyl-2-Propenyl und 1-Ethyl-1-Ethenyl.

In den vorstehend genannten Alkenylgruppen können, soweit nicht anders beschrieben, gegebenenfalls ein oder mehrere Wasserstoffatome durch andere Reste ersetzt sein. Beispielsweise können diese Alkylgruppen durch die Halogenatome Fluor substituiert sein. Es können gegebenenfalls auch alle Wasserstoffatome der Alkenylgruppe ersetzt sein.

Als Alkinylgruppen (auch soweit sie Bestandteil anderer Reste sind) werden verzweigte und unverzweigte Alkinylgruppen mit 2 bis 12 Kohlenstoffatomen bezeichnet, soweit sie mindestens eine Dreifachbindung aufweisen, beispielsweise Ethinyl, Propargyl, Butinyl, Pentinyl, Hexinyl etc., vorzugsweise Ethinyl oder Propinyl.

In den vorstehend genannten Alkinylgruppen können, soweit nicht anders beschrieben, gegebenenfalls ein oder mehrere Wasserstoffatome durch andere Reste ersetzt sein. Beispielsweise können diese Alkylgruppen Fluor substituiert sein. Es können gegebenenfalls auch alle Wasserstoffatome der Alkinylgruppe ersetzt sein.

Der Begriff Aryl steht für ein aromatisches Ringsystem mit 6 bis 14 Kohlenstoffatomen, vorzugsweise 6 oder 10 Kohlenstoffatomen, bevorzugt Phenyl, das, soweit nicht anders beschrieben, beispielsweise einen oder mehrere der nachfolgend genannten Substituenten tragen kann: OH, NO₂ CN, OMe, -OCHF₂, -OCF₃, Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, -COOH, -COO-C₁-C₄-Alkyl, vorzugsweise -O-Methyl oder -O-Ethyl, -CONH₂.

Als Cycloalkylreste werden Cycloalkylreste mit 3 - 12 Kohlenstoffatomen beispielsweise Cyclopropyl, Cyclobutyl, Cyclopentyl, Cyclohexyl, Cycloheptyl oder Cyclooctyl, vorzugsweise Cyclopropyl, Cyclopentyl oder Cyclohexyl bezeichnet, wobei jeder der vorstehend genannten Cycloalkylreste gegebenenfalls ferner einen oder mehrere Substituenten tragen kann, beispielsweise: OH, NO₂, CN, OMe, -OCHF₂, - OCF₃ oder Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, -COOH, -COO-C₁-C₄-Alkyl, vorzugsweise -COO-Methyl oder -COO-Ethyl oder -CONH₂, Besonders bevorzugte Substituenten der Cycloalkylreste sind =O, OH, Methyl oder F.

Als Cycloalkenylreste werden Cycloalkylreste mit 3 - 12 Kohlenstoffatomen, die mindestens eine Doppelbindung aufweisen, beispielsweise Cyclopropenyl, Cyclobutenyl, Cyclopentenyl, Cyclohexenyl oder Cycloheptenyl, vorzugsweise Cyclopropenyl, Cyclopentenyl oder Cyclohexenyl bezeichnet, wobei jeder der vorstehend genannten Cycloalkenylreste gegebenenfalls ferner einen oder mehrere Substituenten tragen kann.

"=O" bedeutet ein über eine Doppelbindung verknüpftes Sauerstoffatom.

Als Heterocycloalkylreste werden, soweit in den Definitionen nicht anders beschrieben, 3 bis 12 gliedrige, vorzugsweise 5- ,6- oder 7-gliedrige, gesättigte oder ungesättigte Heterocyclen, die als Heteroatome Stickstoff, Sauerstoff oder Schwefel enthalten können, beispielsweise Tetrahydrofuran, Tetrahydrofuranon, γ-Butylrolacton, α-Pyran, γ-Pyran, Dioxolan, Tetrahydropyran, Dioxan, Dihydrothiophen, Thiolan, Dithiolan, Pyrrolin, Pyrrolidin, Pyrazolin, Pyrazolidin, Imidazolin, Imidazolidin, Tetrazol, Piperidin, Pyridazin, Pyrimidin, Pyrazin, Piperazin, Triazin, Tetrazin, Morpholin, Thiomorpholin, Diazepan, Oxazin, Tetrahydro-oxazinyl, Isothiazol, Pyrazolidin genannt, vorzugsweise Morpholin, Pyrrolidin, Piperidin oder Piperazin, genannt, wobei der Heterocyclus gegebenenfalls Substituenten tragen kann , beispielsweise C1-C4-Alkyl, vorzugsweise Methyl, Ethyl oder Propyl.

Als Polycycloalkylreste werden gegebenenfalls substituierte, Bi-, Tri-, Tetra- oder pentacyclische Cycloalkylreste, beispielsweise Pinan, 2,2,2-Octan, 2,2,1-Heptan oder Adamantan, bezeichnet. Als Polycycloalkenylreste werden gegebenenfalls verbrückte oder/und substituierte, 8- gliedrige Bi-, Tri-, Tetra- oder pentacyclische Cycloalkenyllreste, vorzugsweise Bicycloalkenyl- oder Tricycloalkenylreste, sofern sie mindestens eine Doppelbindung aufweisen, beispielsweise Norbornen, bezeichnet. Als Spiroalkylreste werden gegebenenfalls substituierte spirocyclische C₅-C₁₂ Alkylreste bezeichnet.

Als Halogen wird im allgemeinen Fluor, Chlor, Brom oder Jod bezeichnet, vorzugsweise Fluor, Chlor oder Brom, besonders bevorzugt Chlor.

Der Substituent R¹ kann ein Rest ausgewählt aus der Gruppe bestehend aus Chlor, Fluor, Brom, Methansulfonyl, Ethansulfonyl, Trifluormethansulfonyl und para-Toluolsulfonyl, vorzugsweise Chlor bedeuten.

Der Substituent R² kann Wasserstoff oder C₁-C₃-Alkyl, bevorzugt Wasserstoff, bedeuten.

Der Substituent R³ kann Wasserstoff,
oder ein Rest ausgewählt aus der Gruppe bestehend aus gegebenenfalls substituiertem C₁-C₁₂-Alkyl, C₂-C₁₂-Alkenyl, C₂-C₁₂-Alkinyl, und C₆-C₁₄-Aryl, vorzugsweise Phenyl,
oder ein Rest ausgewählt aus der Gruppe bestehend aus gegebenenfalls substituiertem und/oder verbrücktem C₃-C₁₂-Cycloalkyl, vorzugsweise Cyclopentyl, C₃-C₁₂-Cycloalkenyl, C₇-C₁₂-Polycycloalkyl, C₇-C₁₂-Polycycloalkenyl, C₅-C₁₂-Spirocycloalkyl und gesättigtes oder ungesättigtes
C₃-C₁₂-Heterocycloalkyl, das 1 bis 2 Heteroatome enthält,
bedeuten.

Die Substituenten R⁴, R⁵ sind gleich oder verschieden und können Wasserstoff,
oder gegebenenfalls substituiertes C₁-C₆-Alkyl,
oder R⁴ und R⁵ gemeinsam eine 2- bis 5-gliedrige Alkylbrücke, die 1 bis 2 Heteroatome enthalten kann,
oder R⁴ und R³ oder R⁵ und R³ gemeinsam eine gesättigte oder ungesättigte C₃-C₄-Alkylbrücke, die gegebenenfalls 1 Heteroatom enthalten kann,
bedeuten.

A₁ und A₂ können -N=, bedeuten.

R⁶ kann einen C₁-C₄-Alkyl, vorzugsweise Methyl oder Ethyl, bedeuten.

Die Herstellung der Verbindung der Formel (II) kann nach literaturbekannten Methoden, beispielsweise analog den in WO 03/020722 beschrieben Synthesen erfolgen.

Die Verbindungen der allgemeinen Formel (I) können u.a. in Analogie zu nachfolgendem Synthesebeispielen synthetisiert werden. Diese Beispiele sind allerdings nur als exemplarische Vorgehensweise zur weitergehenden Erläuterung der Erfindung zu verstehen, ohne selbige auf dessen Gegenstand zu beschränken. Die allgemeine Synthese ist in Schema (1) dargestellt.

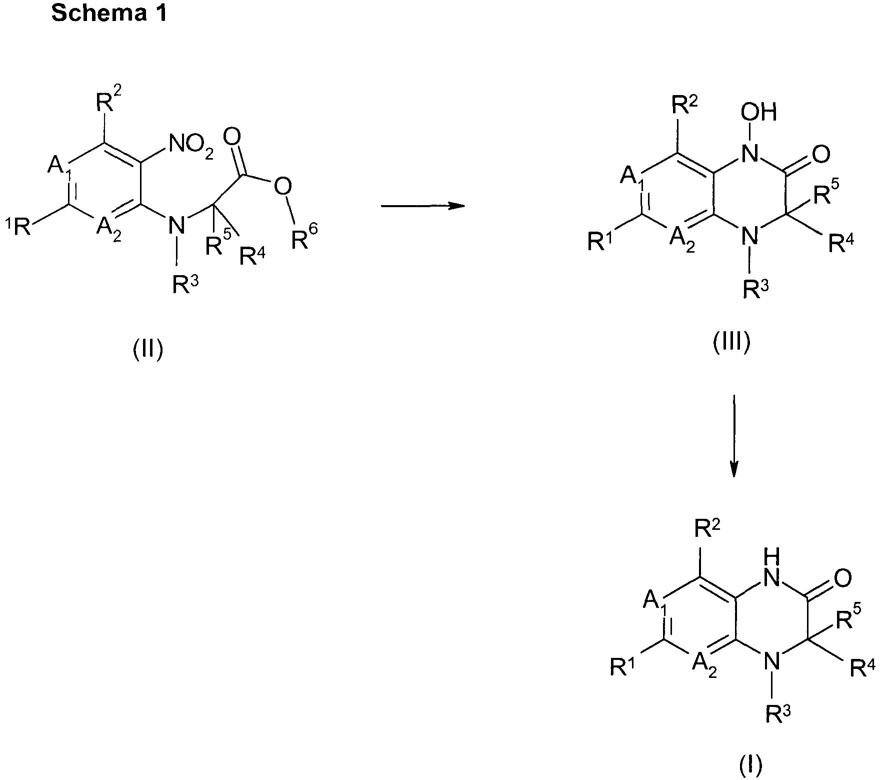

### Synthese von (7R)-2-Chlor-8-cyclopentyl-7-ethyl-5-hydroxy-7,8-dihydro-5H-pteridin-6-on

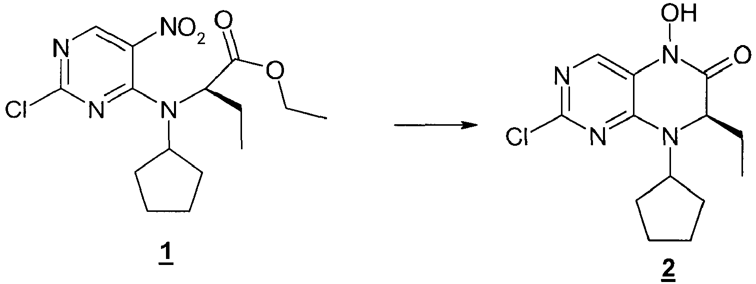

30 g (84.2 mmol) von **1** werden in 300 ml Tetrahydrofuran gelöst und 3 g Pt/C (5%) zugesetzt. Die Reaktionsmischung wird 5 h bei 35°C und einem Wasserstoffdruck von 4 bar hydriert. Der Katalysator wird abfiltriert und mit ca. 30 ml Tetrahydrofuran gewaschen. Das Filtrat wird unter vermindertem Druck eingedampft. Man erhält 25,6g von Produkt **2** als gelben Feststoff.
¹H-NMR (400 MHZ) (DMSO_{d6}): δ 11.05 (bs 1H); 7.85 (s 1H); 4.47-4.45 (dd 1H); 4.16-4.08 (t 1H); 1.95-1.67 (m 10H); 0.80-0.73 (t 3H)

### Synthese von (7R)-2-Chlor-8-cyclopentyl-7-ethyl-7,8-dihydro-5H-pteridin-6-on

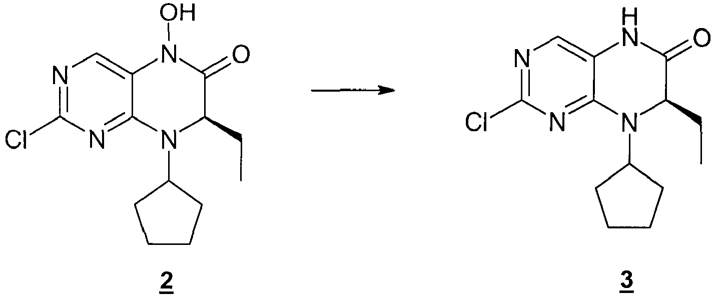

5,22 g (17,6 mmol) von **2** werden in 55 ml Tetrahydrofuran gelöst. 520 mg Pt-C (5%) und 250 mg Vanadium(IV)oxyacetylacetonat werden zugegeben. Die Reaktionsmischung wird 6 Stunden bei 20°C und einem Wasserstoffdruck von 4 bar hydriert. Der Katalysator wird abfiltriert und mit ca. 15 ml Tetrahydrofuran gewaschen. Das Filtrat wird unter vermindertem Druck eingedampft.
Man erhält 5,0 g von Produkt **3** als gelbes Pulver.
¹H-NMR (400 MHz) (DMSO_{d6}): δ 11.82 (bs 1H); 7.57 (s 1H); 4.24-4.21 (dd 1H); 4.17-4.08 (m 1H); 1.97-1.48 (m 10H); 0.80-0.77 (t 3H).

### Synthese von: (7R)-2-Chlor-8-cyclopentyl-7-ethyl-7,8-dihydro-5H-pteridin-6-on

Zu einer Lösung von 700g (1,96 mol) von **1** in 700 ml Tetrahydrofuran gibt man 70g Pt/C (5%). Die Reaktionsmischung wird 2,5 Stunden bei 35°C und einem Wasserstoffdruck von 4 bar bis zum Stillstand der Wasserstoffaufnahme hydriert. Der Autoklav wird geöffnet und man gibt 35g Vanadium(IV)oxyacetylacetonat zu. Man hydriert weitere 2,5 Stunden bei 35°C und einem Wasserstoffdruck von 4 bar. Es wird filtriert und der Rückstand mit Tetrahydrofuran gewaschen. Das Filtrat wird unter vermindertem Druck eingedampft. Der Rückstand wird in 2.75 L Aceton gelöst und durch Zusatz von ebenso viel entmineralisiertem Wasser gefällt. Der Feststoff wird abgenutscht und mit einem Aceton/Wasser Gemisch (1:1), anschließend mit tert.-Butylmethylether gewaschen. Nach Trocknung erhält man 551g von Produkt 3.

### Synthese von: (7R)-2-Chlor-8-cyclopentyl-7-ethyl-7,8-dihydro-5H-pteridin-6-on

30g (84 mmol) von **1** werden in 300 ml Tetrahydrofuran gelöst. 3g Pt/C (5%) und 1,5g Vanadium(IV)oxyacetylacetonat werden zugegeben. Die Reaktionsmischung wird 24 Stunden bei 35°C und einem Wasserstoffdruck von 4 bar bis zur vollständigen Umsetzung hydriert. Es wird filtriert, der Rückstand mit Tetrahydrofuran gewaschen und das Filtrat unter vermindertem Druck eingedampft. Der Rückstand wird in 118 ml Aceton gelöst und durch Zusatz von ebenso viel entmineralisiertem Wasser gefällt. Der Feststoff wird abgenutscht und mit einem Aceton/Wasser Gemisch (1:1) und anschließend mit tert.-Butylmethylether gewaschen. Nach Trocknung erhält man 18g von Produkt 3.

### Synthese von: (7R)-2-Chlor-7-ethyl-8-isopropyl-7,8-dihydro-5H-pteridin-6-on

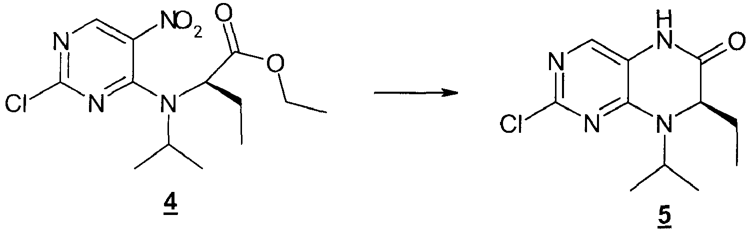

10g (316 mmol) von **4** werden in 800 ml Tetrahydrofuran und 200 ml Isopropanol gelöst. 10g Pt/C (5%) und 5g Vanadium(IV)oxyacetylacetonat werden zugegeben. Die Reaktionsmischung wird 24 Stunden bei 35°C und einem Wasserstoffdruck von 4 bar bis zur vollständigen Umsetzung hydriert. Es wird filtriert und das Filtrat bis zur beginnenden Kristallisation eingedampft. 150 ml Isopropanol werden zugegeben und die Suspension bis zur vollständigen Lösung auf 70-80°C erwärmt. Nach Zugabe von 600 ml entmineralisiertem Wasser wird das Produkt zur Kristallisation gebracht. Es wird abgesaugt und mit entmineralisiertem Wasser gewaschen. Nach Trocknung erhält man 68g von Produkt **5**.
¹H-NMR (400 MHz) (DMSO_{d6}): δ 10.81 (bs 1H); 7.56 (s 1H); 4.37-4.24 (m 2H); 1.89-1.65 (m 2H); 1.34-1.31 (m 6H); 0.80-0.73 (t 3H)

## Patentansprüche

1. Verbindungen der Formel (III) 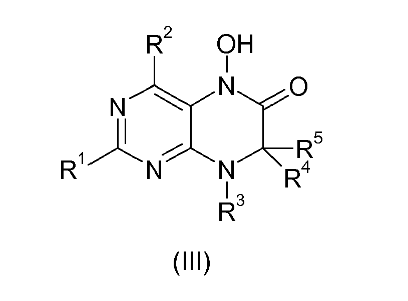 worin
R¹ ein Rest ausgewählt aus der Gruppe bestehend aus Chlor
R² Wasserstoff,
R³ Wasserstoff oder ein Rest ausgewählt aus der Gruppe bestehend aus C₁-C₄-Alkyl, Cyclopropyl, Cyclopentyl und Cyclohexyl
R⁴, R⁵ gleich oder verschieden, Wasserstoff oder gegebenenfalls substituiertes C₁-C₄-Alkyl,

2. Verbindung der Formel **2**

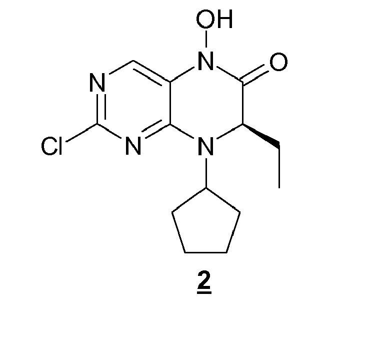

## Claims

1. Compounds of formula (III) 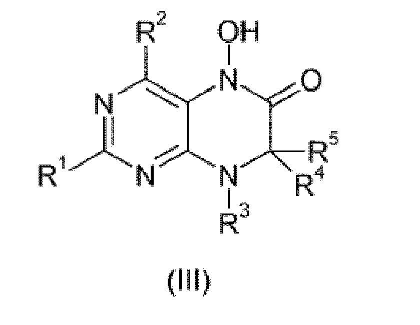 wherein
R¹ denotes a group selected from the group consisting of chlorine
R² denotes hydrogen,
R³ denotes hydrogen or a group selected from the group consisting of C₁-C₄-alkyl, cyclopropyl, cyclopentyl and cyclohexyl,
R⁴, R⁵ which may be identical or different denote hydrogen or optionally substituted C₁-C₄-alkyl,

2. Compound of formula **2**

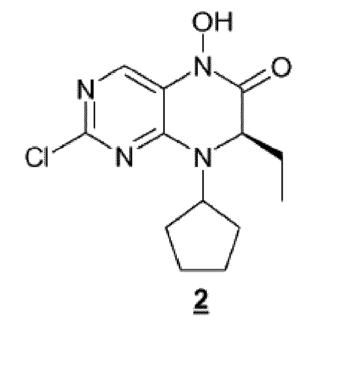

## Revendications

1. Composés de formule (III) 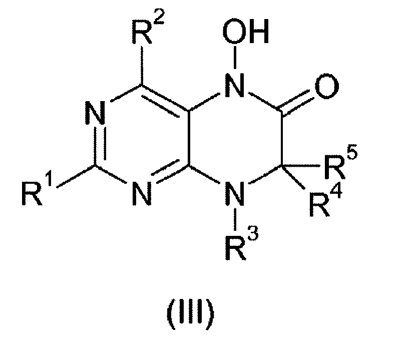 dans laquelle
R¹ représente un radical choisi dans le groupe comprenant un atome de chlore
R² représente un atome d'hydrogène,
R³ représente un atome d'hydrogène ou un radical choisi dans le groupe comprenant les groupes alkyle en C₁ à C₄, cyclopropyle, cyclopentyle et cyclohexyle
R⁴, R⁵, identiques ou différents, représentent un atome d'hydrogène ou un groupe alkyle en C₁ à C₄ éventuellement substitué.

2. Composé de formule 2
